# EUROPÄISCHE PATENTSCHRIFT

(11) **EP 2 217 555 B1**
(45) Veröffentlichungstag und Bekanntmachung des Hinweises auf die Patenterteilung: **09.09.2015**
(21) Anmeldenummer: 08848555.2
(22) Anmeldetag: 28.10.2008
(51) Int. Cl.: C07C 45/50, C07C 47/32, C07C 47/02

(54) **VERFAHREN ZUR HERSTELLUNG VON ALDEHYDEN**
METHOD FOR THE PRODUCTION OF ALDEHYDES
PROCÉDÉ DE PRODUCTION D'ALDÉHYDES

(30) Priorität: 09.11.2007 DE 102007053385
(43) Veröffentlichungstag der Anmeldung: 18.08.2010
(73) Patentinhaber: OXEA GmbH, 46147 Oberhausen (DE)
(72) Erfinder: FISCHBACH, Andreas, 84419 Schwindegg (DE); SCHMID, Klaus, 46539 Dinslaken (DE); BALZAREK, Christoph, 47903 Krefeld (DE); DUKAT, Wolfgang, 46145 Oberhausen (DE); FÜRMEIER, Sandra, 34327 Körle (DE); RAINER, Lukas, 45133 Essen (DE); SCHOLZ, Horst, 46435 Dinslaken (DE); STORM, Edgar, 46147 Oberhausen (DE)
(86) Internationale Anmeldenummer: PCT/EP2008/009084
(87) Internationale Veröffentlichungsnummer: WO 2009/059713

(56) Entgegenhaltungen:
- DE-A1- 1 920 960
- DE-A1- 3 822 038

## Beschreibung

Die vorliegende Erfindung betrifft ein Verfahren zur Herstellung von Aldehyden in Gegenwart eines Katalysators auf der Basis von Rhodium und in Abwesenheit von komplexbildenden Organophosphor-Verbindungen in einer Reaktionszone, wobei man der Reaktionszone eine Rhodium haltige organische Lösung zuführt, die zuvor mit mindestens einer organischen Säure oder Mischungen davon versetzt wird.

Als Hydroformylierung oder Oxo-Reaktion bezeichnet man die übergangsmetallkatalysierte Umsetzung von Olefinen oder olefinisch ungesättigten Verbindungen mit Wasserstoff und Kohlenmonoxid zu Aldehyden und Alkoholen, die ein Kohlenstoffatom mehr als das eingesetzte Olefin enthalten. Der Hydroformylierungsprozess hat mittlerweile erhebliche wirtschaftliche und technische Bedeutung erlangt. Die dabei primär erhaltenen Aldehyde werden als solche verwendet oder stellen wertvolle Vorprodukte für die Gewinnung von beispielsweise Alkoholen, Carbonsäuren, Estern oder Aminen dar.

Die Hydroformylierung wird durch Hydridometallcarbonyle, vorzugsweise solcher der Metalle der VIII. Nebengruppe des Periodensystems der Elemente, katalysiert. Neben Kobalt, dem klassischen Katalysatormetall, werden seit einigen Jahren zunehmend Katalysatoren auf Basis von Rhodium eingesetzt. Im Gegensatz zu Kobalt gestattet es Rhodium, die Reaktion bei niedrigerem Druck durchzuführen. Darüber hinaus werden bei Einsatz endständiger Olefine bevorzugt geradkettige n-Aldehyde und nur in untergeordnetem Maße iso-Aldehyde gebildet. Schließlich ist auch die Hydrierung der eingesetzten Olefine zu gesättigten Kohlenwasserstoffen in Gegenwart von Rhodium-Katalysatoren deutlich niedriger als bei Anwendung von Kobalt-Katalysatoren.

Industriell wird die Hydroformylierung olefinisch ungesättigter Verbindungen unter der katalytischen Wirkung von Rhodiumcarbonyl-Komplexen mit tertiären organischen Phosphinen oder Phosphiten als Liganden durchgeführt. Bei einer Prozessvariante arbeitet man in homogener Phase, d.h., eingesetztes Olefin, Katalysator und Reaktionsprodukte liegen gemeinsam in Lösung vor. Die Reaktionsprodukte werden aus dem Gemisch meist destillativ, seltener nach anderen Verfahren wie Extraktion, abgetrennt. Das in homogener Phase durchgeführte Hydroformylierungsverfahren kann in Form eines Gaskreislaufverfahrens gemäß US 4,247,486 oder in Form eines Flüssigkeitskreislaufverfahrens gemäß US 4,148,830 ausgestaltet werden.

Nach einer weiteren Verfahrensvariante kann die Rhodium katalysierte Hydroformylierungsreaktion auch in Abwesenheit von komplexbildenden Liganden, beispielsweise von Phosphinen oder Phosphiten betrieben werden. Solche, nicht mit Phosphinen oder Phosphiten modifizierten Rhodiumkatalysatoren und ihre Eignung als Hydroformylierungskatalysatoren sind aus der Literatur her bekannt und sie werden als unmodifizierte Rhodiumkatalysatoren bezeichnet. Es wird in der Fachliteratur angenommen, dass die Rhodiumverbindung HRh(CO)₄ die katalytisch aktive Rhodiumspezies bei der Hydroformylierung mit unmodifizierten Rhodiumkatalysatoren ist, obgleich dies aufgrund der vielen in der Reaktionszone nebeneinander ablaufenden Chemismen nicht eindeutig bewiesen ist. Die unmodifizierten Rhodiumkatalysatoren bilden sich unter den Bedingungen der Hydroformylierungsreaktion in der Reaktionszone aus Rhodiumverbindungen, beispielsweise Rhodiumsalzen, wie Rhodium(III)chlorid, Rhodium(III)nitrat, Rhodium(III)acetat, Rhodium(II)acetat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise Rhodaten, aus Rhodiumcarbonylverbindungen, wie Rh₄(CO)₁₂ und Rh₆(CO)₁₆ oder aus Organorhodiumverbindungen, wie Rhodiumcarbonylacetonylacetonat, Cyclooctadienrhodiumacetat oder -chlorid in Gegenwart von Kohlenmonoxid/Wasserstoffgemischen, die man auch als Synthesegas bezeichnet. Dabei kann die Rhodiumverbindung als Feststoff oder zweckmäßigerweise in Lösung eingesetzt werden. Verfahren zur Hydroformylierung in Gegenwart von unmodifizierten Rhodiumkomplexen sind beispielsweise aus der DE 38 22 038 A1, wobei Rhodium-2-ethylhexanoat eingesetzt wird, oder aus der EP 0 695 734 A1 bekannt, nach der eine Lösung einer zuvor gebildeten Rhodiumcarbonylverbindung in der umzusetzenden olefinisch ungesättigten Verbindung verwendet wird.

Infolge des Fehlens von stabilisierenden Liganden kann es bei der destillativen Aufarbeitung des Hydroformylierungsrohgemisches, das nach der unmodifizierten Verfahrensvariante erhalten und der Reaktionszone entnommen wurde, zur Abscheidung von metallischem Rhodium kommen. Das in der Aufarbeitungsapparatur des Hydroformylierungsrohgemisches ausgefallene Rhodium kann nicht wieder in den Hydroformylierungsprozess zurückgeführt werden und führt daher zu Rhodiumverlusten, die aufgrund der hohen Edelmetallkosten einen wirtschaftlichen Nachteil bedeuten. Um Rhodiumverluste in der Aufarbeitungsstufe eines rohen Hydroformylierungsgemisches, das nach der unmodifizierten Rhodiumhydroformylierung erhalten wurden, zu verringern, schlägt EP 0 695 734 A1 zunächst eine Extraktion mit einer wässrigen Lösung eines wasserlöslichen phosphorhaltigen Komplexbildners vor, wobei Rhodium in die wässrige Phase extrahiert wird und wobei aus dem verbliebenen Hydroformylierungsaustrag Aldehyd oder Alkohol isoliert wird. Anschließend wird der wässrige Extrakt mit einer organischen Flüssigkeit in Gegenwart von Kohlenmonoxid oder Kohlenmonoxid haltigen Gasen unter Druck und bei erhöhter Temperatur behandelt, wobei Rhodium als Rhodiumcarbonyl wieder in die organische Phase übergeht, die anschließend wieder der Reaktionszone zugeführt werden kann.

Neben den Rhodiumabscheidungen, die bei der Aufarbeitung eines rohen, nach der unmodifizierten Rhodiumkatalyse erhaltenen Hydroformylierungsgemisches auftreten können, ist auch beim Einleiten der Rhodium haltigen Lösung in der Reaktionszone mit der Abscheidung von Rhodiummetall nahe des Einleitbereiches zu rechnen. Dieses abgeschiedene Rhodiummetall wandelt sich in der Reaktionszone selbst unter Synthesegasdruck nicht mehr in katalytisch aktives Rhodiumcarbonyl um. Daher steht, bezogen auf den Rhodiumeinsatz, zum einen nur eine geringere Menge an katalytisch aktivem Rhodium zur Verfügung und zum anderen verbleibt das abgeschiedene Rhodiummetall in der Reaktionszone und führt zu Rhodiumverlusten. Nach der DE 19 20 960 A1 ist bei einem kontinuierlich betriebenen, unmodifizierten Hydroformylierungsprozess das Erhitzen der Rhodiumlösung sowie der Mischung aus olefinisch ungesättigter Verbindung und Rhodiumlösung in Abwesenheit von Kohlenmonoxid zu vermeiden. Ebenfalls sollen beim Mischen der Rhodiumlösung mit der olefinisch ungesättigten Verbindung in der Reaktionszone keine hohen Rhodiumkonzentrationen auftreten. DE 19 20 960 A1 empfiehlt daher, Synthesegas, die olefinisch ungesättigte Verbindung und die Rhodiumlösung in der Nähe der Einführung in der Reaktionszone innig zu durchmischen. Dabei wird eine Lösung einer Rhodiumverbindung, beispielsweise von Rhodiumchlorid oder -nitrat, in einem polaren organischen Lösungsmittel der Reaktionszone zugeführt, wobei die Rhodiumverbindung in der olefinisch ungesättigten Verbindung unlöslich sein soll. Beispielsweise wird eine Lösung von Rhodiumacetat in einer Mischung aus Methanol und Essigsäure, in Essigsäure oder in Propionsäure eingesetzt. Der bekannte kontinuierlich betriebene Hydroformylierungsprozess erlaubt Umsatzraten für 1-Octen von 94 - 96%, bei einer Verweilzeit von etwa 2 Stunden und bei einer Rhodiumkonzentration von 14 ppm, wobei gleichzeitig Rhodiumabscheidungen in der Nähe des Einleitens der Rhodiumlösung in der Reaktionszone reduziert werden können. Es wird ebenfalls darauf hingewiesen, dass die zugesetzte Rhodiumverbindung in der olefinisch ungesättigten Verbindung unlöslich oder nahezu unlöslich sein soll, um hohe Rhodiumkonzentrationen bei dem Vermischen der Rhodiumlösung mit der olefinisch ungesättigten Verbindung zu vermeiden. Bei lokal auftretenden hohen Rhodiumkonzentrationen kann es in Gegenwart der olefinisch ungesättigten Verbindung zur Abscheidung von Rhodiummetall kommen.

Überraschenderweise wurde gefunden, dass ein gezielter Zusatz einer organischen Säure zu einer Rhodium haltigen organischen Lösung, die anschließend der Reaktionszone zugeführt wird, in einem unmodifizierten Rhodium katalysierten Hydroformylierungsprozess zu einer deutlichen Umsatzsteigerung bei gleich bleibend hoher Selektivität führt, so dass durch den gezielten Säurezusatz die Ausbeute an der gewünschten aldehydischen Verbindung erhöht werden kann. Soll hingegen aufgrund der Anlagenauslegung oder aufgrund von Marktgegebenheiten die absolute Ausbringung an dem gewünschten Aldehyd gleichbleiben, so kann gemäß der erfindungsgemäßen Arbeitsweise der spezifische Rhodiumeinsatz, bezogen auf die hergestellte Aldehydmenge, reduziert werden, was einen erheblichen wirtschaftlichen Vorteil bedeutet.

Die Erfindung besteht daher in einem Verfahren zur Herstellung von Aldehyden durch Zuführen einer organischen Lösung, die mindestens eine Rhodiumverbindung gelöst enthält, zu einer Reaktionszone und Umsetzung von olefinisch ungesättigten Verbindungen mit Kohlenmonoxid und Wasserstoff in dieser Reaktionszone in homogener organischer Phase in Gegenwart mindestens einer Rhodiumverbindung und in Abwesenheit von komplexbildenden Organophosphor-Verbindungen. Es ist dadurch gekennzeichnet, dass man der Rhodium haltigen organischen Lösung je Mol Rhodium mindestens eine organische Säure ausgewählt von gesättigten aliphatischen Monocarbonsäuren mit 5 bis 13 Kohlenstoffatomen im Molekül, gesättigten aliphatischen Dicarbonsäuren mit 5 bis 13 Kohlenstoffatomen im Molekül und Sulfonsäuren mit 1 bis 12 Kohlenstoffatomen im Molekül oder Mischungen davon auf einen Gesamtgehalt, berechnet als Säureäquivalent, von mehr als 3 Mol bis 3000 Mol zusetzt und anschließend diese Lösung der Reaktionszone zuführt.
Die der Reaktionszone zugeführte organische Lösung, die mindestens eine Rhodiumverbindung gelöst enthält, wird im Folgenden auch als Rhodiumlösung bezeichnet. Die Lösungsmittel, die zur Zubereitung dieser Rhodiumlösung eingesetzt werden, haben ein vollständiges Auflösen der Rhodiumver-bindung sicherzustellen, Geeignete Lösungsmittel sind beispielsweise wasserunlösliche Ketone, Dialkylether, aliphatische Nitrile, aromatische Kohlenwasserstoffe wie Benzol oder Toluol, die isomeren Xylole oder Mesitylen, gesättigte cycloaliphatische Kohlenwasserstoffe wie Cyclopentan oder Cyclohexan oder gesättigte aliphatische Kohlenwasserstoffe, wie n-Hexan, n-Heptan oder n-Octan. Auch polare Lösungsmittel, wie aliphatische gesättigte Monoalkohole, beispielsweise, Methanol, Ethanol, Propanol, n- und iso-Butanol, die isomeren Pentanole, 2-Ethylhexanol, Isononanol auf Basis von 3,5,5-Trimethylhexanol, gesättigte mehrwertige Alkohole, wie 1,3-Propandiol, 1,4-Butandiol oder Polyalkylenglykole, wie Ethylenglykol, Triethylenglykol und deren Mono- und Diether, beispielsweise deren Methyl- oder Butylether, oder polare Esterverbindungen wie Texanol® der Firma Eastman sind geeignet. Auch Mischungen von Lösungsmitteln können eingesetzt werden, solange eine ausreichende Löslichkeit der Rhodiumverbindung sichergestellt ist und sich das Lösungsmittel oder das Lösungsmittelgemisch unter den nachfolgenden Hydroformylierungsbedingungen als inert erweist. Als besonders geeignet haben sich 2-Ethylhexanol oder Toluol als Lösungsmittel zur Zubereitung der Rhodiumlösung erwiesen.

Als Rhodiumverbindungen, die zur Herstellung der Rhodiumlösung eingesetzt werden, verwendet man Salze aliphatischer Mono- oder Polycarbonsäuren mit 2 bis 13, vorzugsweise 7 bis 11 Kohlenstoffatomen im Molekül. Geeignete Rhodiumsalze sind Rhodiumacetat, Rhodiumpropionat, Rhodiumoxalat, Rhodiummalonat, Rhodium-2-ethylhexanoat, Rhodium-isononanoat, wobei die Isononansäure durch Hydroformylierung von Diisobutylen mit nachfolgender Oxidation des Hydroformylierungsproduktes hergestellt wird, oder Rhodium-isotridecanoat, wobei die Isotridecansäure durch Hydroformylierung von Tetrapropylen und nachfolgende Oxidation des Hydroformylierungsproduktes hergestellt wird. Weiterhin haben sich Carbonylverbindungen des Rhodiums wie Tricarbonylrhodium, Rh(CO)₃, Tetracarbonylrhodium, [Rh(CO)₄]₂, Tetrarhodiumdodekacarbonyl Rh₄(CO)₁₂ sehr bewährt. Halogencarbonylverbindungen wie Dicarbonylrhodiumbromid, [Rh(CO)₂]Br und Dicarbonylrhodiumjodid [Rh(CO)₂]I, können zwar auch eingesetzt werden, finden wegen des korrosiven Verhaltens der Halogenionen jedoch nur begrenzte Anwendung. Schließlich sind auch komplexe Verbindungen des Rhodiums, insbesondere Rhodium(III)-Verbindungen, geeignete Ausgangsmaterialien zur Herstellung der katalytisch aktiven Metallkomponente im Katalysatorsystem. Diese Verbindungen enthalten ein-, zwei- oder dreibindige Liganden wie β-Diketone, z. B. Acetylaceton, oder aliphatische oder cycloaliphatische und diethylenisch ungesättigte Kohlenwasserstoffe wie Cyclopentadien und 1,5-Cyclooctadien. Für die Zubereitung der Rhodiumlösung besonders geeignete Rhodiumverbindungen sind die Rhodiumoxide, die Rhodiumcarbonyle, Rhodiumacetat, Rhodium-2-ethylhexanoat, Rhodiumisononanoat und Rhodium(III)-acetylacetonat.

Die Herstellung der Rhodiumlösung aus der Rhodiumverbindung und dem Lösungsmittel oder Lösungsmittelgemisch erfolgt in konventioneller Arbeitsweise, beispielsweise durch einfaches Auflösen der frischen Rhodiumverbindung, wobei der Ausschluss von Sauerstoff zu empfehlen ist. Darüber hinaus können auch solche Rhodiumlösungen eingesetzt werden, die bei der Aufarbeitung von gebrauchten Rhodiumkatalysatoren anfallen. Derartige Rhodium haltige organische Lösungen, in denen Rhodiumcarboxylate gelöst vorliegen, können nach den Verfahren gemäß DE 36 26 536 A1 oder DE 38 33 427 A1 gewonnen werden. Dabei wird zunächst eine wässrige Rhodiumlösung in Gegenwart eines Carboxylates oder einer Mischung aus einem Carboxylat und einer Carbonsäure, beispielsweise 2-Ethylhexansäure, oxidiert. Rhodium fällt als wasserunlösliches Carboxylat an, das mit einem wasserunlöslichen Lösungsmittel, beispielsweise Toluol, in die organische Phase extrahiert werden kann. Dabei kann angenommen werden, dass auch die im vorgeschalteten Oxidationsschritt anwesende Carbonsäure sich in dem organischen Lösungsmittel löst. Die nach DE 36 26 536 A1 und DE 38 33 427 A1 erhaltenen Rhodiumlösungen weisen daher schon einen bestimmten Carbonsäureanteil auf und werden nach dem Stand der Technik ohne Nachbehandlung als Katalysatorbestandteil für die Hydroformylierungsreaktion eingesetzt.

Überraschenderweise wurde gefunden, dass bei Rhodiumlösungen, die bereits eine bestimmte Menge an einer Carbonsäure gelöst enthalten, sich der aktive Zusatz einer organische Säure auf die Aldehydausbeute in der nachgeschaltenen Hydroformylierungsreaktion als vorteilhaft erweist. Erfindungsgemäß wird der Rhodiumlösung eine solche Menge mindestens einer organischen Säure zugesetzt, dass je Mol Rhodium mehr als 3 Mol bis 3000 Mol, vorzugsweise 50 bis 2000 Mol und insbesondere 100 bis 1000 Mol organische Säure, berechnet als Säureäquivalent, vorliegen. Bezogen auf die Rhodiummenge kann der gezielte Zusatz an organischer Säure überraschenderweise über einen weiten Bereich variiert werden, ohne dass es zur nennenswerten Bildung unerwünschter Nebenprodukte, beispielsweise von Hochsiedern, kommt. Überschreitet man jedoch das kritische Molverhältnis, wird der Säuregehalt im Reaktionsgemisch zu hoch, so dass mit Nebenreaktionen mit den bereits gebildeten Aldehyden zu rechnen ist. Ebenfalls verteuert ein zu hoher Säurezusatz unnötig das Hydroformylierungsverfahren. Wird hingegen das kritische Molverhältnis Rhodium zu organischer Säure unterschritten, beobachtet man keinen vorteilhaften Einfluss mehr auf die Aldehydausbeute Die Zugabe der organischen Säure zu der Rhodiumlösung erfolgt im Allgemeinen bei Raumtemperatur unter konventionellen Bedingungen. Man kann unter Ausschluss von Sauerstoff arbeiten, was jedoch nicht zwingend erforderlich ist.

Als organische Säuren eignen sich gesättigte aliphatische Mono- und Dicarbonsäuren mit 5 bis 13, vorzugsweise 5 bis 11 Kohlenstoffatomen im Molekül. Beispielhaft können n-Valeriansäure, 2-Methylbuttersäure, 2-Ethylhexansäure, Isononansäure, hergestellt durch Hydroformylierung von Diisobutylen und nachfolgende Oxidation des Hydroformylierungsproduktes, oder Isotridecansäure, hergestellt durch Hydroformylierung von Tetrapropylen und nachfolgende Oxidation des Hydroformylierungsproduktes, auf den geforderten Gehalt zugesetzt werden. Als geeignete aliphatische Dicarbonsäuren kann Adipinsäure zugesetzt werden. Zu den organischen Säuren, die mit Erfolg in dem erfindungsgemäßen Verfahren eingesetzt werden können, zählen ebenfalls Sulfonsäuren mit 1 bis 12 Kohlenstoffatomen im Molekül , insbesondere aliphatische, cycloaliphatische, aromatische und/oder araliphatische Sulfonsäuren, beispielsweise Methansulfonsäure, para-Toluolsulfonsäure, Benzolsulfonsäure oder Benzoldisulfonsäure. Besonders bewährt hat sich der Zusatz von verzweigten gesättigten aliphatischen Monocarbonsäuren wie 2-Ethylhexansäure oder Isononansäure bei der Zubereitung der Rhodiumlösung. Auch Gemische von organischen Säuren, beispielsweise Gemische aus 2-Ethylhexansäure und Isononansäure, können verwendet werden. Bei dem Zusatz eines Gemisches von organischen Säuren zu der Rhodiumlösung auf den gewünschten Gehalt bezieht sich das zuvor genannte Molverhältnis von Rhodium zu organischer Säure auf den Gesamtgehalt an zugesetzten organischen Säuren, berechnet als Säureäquivalent.
Die Rhodiumkonzentration in der Rhodiumlösung, die nach Zusatz der organischen Säure der Reaktionszone zugeführt wird, ist im Vergleich zu den Verhältnissen in der Reaktionszone selbst, in der die Hydroformylierungsreaktion stattfindet, vergleichsweise hoch und beträgt im Allgemeinen 100 bis 10000 ppm, vorzugsweise 1000 bis 10000 ppm. Nach dem Einleiten der Rhodiumlösung in die Reaktionszone erniedrigt sich die Rhodiumkonzentration infolge der Verdünnung in der zugesetzten olefinisch ungesättigten Verbindung und in dem in der Hydroformylierungsstufe gegebenenfalls anwesenden Lösungsmittel auf einen Gehalt von 1 bis 100 ppm. Unter den in der Reaktionszone herrschenden Bedingungen bildet sich dann aus der in der eingeleiteten Rhodiumlösung gelösten Rhodiumverbindung in Gegenwart von Synthesegas der eigentliche aktive Hydroformylierungskatalysator.

Das erfindungsgemäße Hydroformylierungsverfahren wird in homogener organischer Phase in Gegenwart mindestens einer Rhodiumverbindung und in Abwesenheit von komplexbildenden Organophosphor-Verbindungen durchgeführt. Solche, beispielsweise nicht mit Phosphinen oder Phosphiten modifizierte Rhodiumkatalysatoren und ihre Eignung als Katalysator zur Hydroformylierung sind aus der Literatur bekannt und sie werden als unmodifizierte Rhodiumkatalysatoren bezeichnet. Es wird in der Fachliteratur angenommen, dass die Rhodiumverbindung HRh(CO)₄ die katalytisch aktive Rhodiumspezies bei der Hydroformylierung mit unmodifizierten Rhodiumkatalysatoren ist. Da im Allgemeinen die Verwendung von nicht mit Phosphinen modifizierten Rhodiumkatalysatoren einen geringeren Rhodiumgehalt erfordert, arbeitet man im Allgemeinen mit einem Rhodiumgehalt von 1 bis 100 ppm, vorzugsweise von 2 bis 30 ppm, bezogen auf das homogene Reaktionsgemisch.

Der Begriff homogene organische Phase steht für eine im Wesentlichen aus dem Lösungsmittel, falls in der Hydroformylierungsstufe zugesetzt, dem Katalysator, der in der eingeleiteten Rhodiumlösung gelösten Rhodiumverbindung einschließlich des vorhandenen Lösungsmittels und der zugesetzten organischen Säure, der unumgesetzten olefinisch ungesättigten Ausgangsverbindung, dem gebildeten Aldehyd sowie den gebildeten Nebenprodukten zusammengesetzte homogene Lösung. Gegebenenfalls kann sich ein Lösungsmittelzusatz in der Hydroformylierungsstufe als zweckmäßig erweisen. Als Lösungsmittel für die Hydroformylierungsreaktion werden organische Verbindungen eingesetzt, in denen Ausgangsmaterial, einschließlich der über die Rhodiumlösung zugeführten Rhodiumverbindung, des Lösungsmittel und der organischen Säure, Reaktionsprodukt und Katalysator löslich sind. Beispiele für solche Lösungsmittel sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofouran, Ketone oder Texanol ® der Firma Eastman. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Die Umsetzung der Olefine bzw. der olefinisch ungesättigten Verbindungen mit Wasserstoff und Kohlenmonoxid zu Aldehyden erfolgt bei Temperaturen von 20 bis 180°C, bevorzugt 50 bis 150°C und insbesondere 100 bis 150°C und Drücken von 0,1 bis 70 MPa, vorzugsweise 0,1 bis 60 MPa und insbesondere 0,1 bis 30 MPa. Die im Einzelfall anzuwendenden Reaktionsbedingungen hängen auch von der Art der umzusetzenden olefinisch ungesättigten Verbindung ab. So lassen sich reaktionsfähige Einsatzstoffe bereits bei relativ niedrigen Temperaturen und Drücken und in Gegenwart geringer Katalysatormengen umsetzen, während reaktionsträgere Verbindungen entsprechend energischere Reaktionsbedingungen erfordern.

Die Zusammensetzung des Synthesegases, d. h. die Anteile von Kohlenmonoxid und Wasserstoff im Gasgemisch, kann in weiten Grenzen variiert werden. Im Allgemeinen setzt man Gemische ein, in denen das Molverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab.
Die olefinische Verbindung kann als solche oder in Lösung der Reaktionszone zugeführt werden. Geeignete Lösungsmittel sind Ketone wie Aceton, Methylethylketon, Acetophenon, niedrigere aliphatische Nitrile wie Acetonitril, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cyclohexan.

Das erfindungsgemäße Verfahren kann sowohl diskontinuierlich als auch kontinuierlich durchgeführt werden. Die Gewinnung der gewünschten Aldehyde aus dem rohen Hydroformylierungsprodukt erfolgt nach konventionellen Verfahren, beispielsweise durch Destillation. Aldehyd und weitere flüchtige Komponenten werden als Kopfprodukt abgezogen und, bei Bedarf, einer weiteren Feinreinigung unterzogen. Die eingesetzten Rhodiummengen fallen im Destillationsrückstand an und werden nach bekannten Verfahren zurückgewonnen.

Die Anwendung des neuen Verfahrens ist nicht auf bestimmte olefinisch ungesättigte Verbindungen als Ausgangsstoffe beschränkt. Die olefinisch ungesättigte Verbindung kann eine oder mehr als eine Kohlenstoff-Kohlenstoff-Doppelbindung enthalten. Die Kohlenstoff-Kohlenstoff-Doppelbindung kann endständig oder innenständig (interne Olefine) angeordnet sein. Bevorzugt sind olefinisch ungesättigte Verbindungen mit endständiger Kohlenstoff-Kohlenstoff-Doppelbindung oder cyclische olefinisch ungesättigte Verbindungen mit unsubstituierten Doppelbindungen.

Beispiele für α-olefinische Verbindungen (mit endständiger Kohlenstoff-Kohlenstoff-Doppelbindung) sind Alkene, Alkylenalkanoate, Alkylenalkylether und Alkenole, insbesondere solche mit 2 bis 12 Kohlenstoffatomen. Ohne Anspruch auf Vollständigkeit seien als α-olefinische Verbindungen Ethylen, Propylen, 1-Buten, 1-Penten, 1-Hexen, 1-Hepten, 1-Octen, 1-Decen, 1-Dodecen, 1-Octadecen, 2-Ethyl-1-hexen, Styrol, 3-Phenyl-1-propen, Allylchlorid, 1,4-Hexadien, 1,3-Butadien, 1,7-Octadien, 3-Cyclohexyl-1-buten, Hex-1-en4-ol, Oct-1-en-4-ol, Vinylcyclohexen, n-Propyl-7-octenoat, 7-Octensäure, 5-Hexenamid genannt.

Als Beispiele weiterer geeigneter olefinischer Verbindungen seien Buten-2, Diisobutylen, Tripropylen, Raffinat II (Gemisch aus 1-Buten, 2-Buten und Butan), Octol oder Dimersol (Dimerisierungsprodukte von Butenen), Tetrapropylen, Cyclohexen, Dicyclopentadien, acyclische, cyclische oder bicyclische Terpene, wie Myrcen, Limonen und Pinen erwähnt. Die Hydroformylierung wird bevorzugt mit olefinisch ungesättigten Verbindungen mit 2 bis 12 Kohlenstoffatomen im Molekül durchgeführt.

Im Folgenden wird das erfindungsgemäße Verfahren anhand einiger Beispiele näher erläutert, es ist jedoch nicht auf die beschriebenen Ausführungsformen beschränkt.

### Beispiele

### Allgemeine Arbeitsvorschrift

In einem 1 L Edelstahlautoklaven wurden 300 g Olefin innerhalb von 30 Minuten auf 130 °C erhitzt und mit einem Synthesegasdruck (Molverhältnis CO/H₂ = 1:1) von 23 MPa beaufschlagt. Nach Erreichen der Reaktionstemperatur wurde die zuvor in eine externe Vorlage eingefüllte Rhodiumlösung spontan mittels eines Synthesegasdifferenzdrucks von 3 MPa in den Autoklaven gedrückt. Die Hydroformylierung wurde für mindestens 2 h bei 130 °C und 26 MPa durchgeführt. Proben wurden nach 30 und 60 Minuten gezogen und gaschromatographisch analysiert. Nach 120 Minuten Reaktionszeit lagen die Umsätze an Cyclohexen bzw. 1-Hexen bei über 98%.

### Beispiel 1 (Vergleichsbeispiel)

### Hydroformylierung von Cyclohexen

Entsprechend der allgemeinen Versuchsvorschrift wurde Cyclohexen in Gegenwart von 3 ppm Rhodium in Form von Rhodium-2-Ethylhexanoat zur Reaktion gebracht. In der über die externe Vorlage zugeführten Rhodiumlösung mit 2-Ethylhexanol als Lösungsmittel lag ein molares Verhältnis von Rhodium zu 2-Ethylhexansäure von 1 zu 3 vor. Weitere 2-Ethylhexansäure wurde der Rhodiumlösung vor dem Einleiten in den Autoklaven nicht zugesetzt. Nach 60 Minuten lag der Cyclohexenumsatz bei 70,4%, gaschromatographisch ermittelt. Die Selektivität zu Formylcyclohexan lag bei 94,7%, gaschromatographisch ermittelt und entspricht einer Ausbeute von 66,7%, gaschromatographisch ermittelt.
Die spezifische Katalysatoraktivität lag bei 26,47 mol Olefin · (mmol Rh · h)⁻¹. Dieser Wert kann als Referenzwert mit "100" angesetzt werden.

### Beispiel 2

### Hydroformylierung von Cyclohexen mit Zusatz von 2-Ethylhexansäure

Entsprechend der allgemeinen Versuchsvorschrift wurde Cyclohexen in Gegenwart von 3 ppm Rhodium in Form von Rhodium-2-Ethylhexanoat zur Reaktion gebracht. In der über die externe Vorlage zugeführten Rhodiumlösung mit 2-Ethylhexanol als Lösungsmittel wurde vor dem Einleiten in den Autoklaven durch Zugabe von 2-Ethylhexansäure ein molares Verhältnis von Rhodium zu 2-Ethylhexansäure von 1:125 eingestellt. Nach 60 Minuten lag der Cyclohexenumsatz bei 72,0%, gaschromatographisch ermittelt. Die Selektivität zu Formylcyclohexan lag bei 95,7%, gaschromatographisch ermittelt und entspricht einer Ausbeute von 68,9%, gaschromatographisch ermittelt.
Die spezifische Katalysatoraktivität lag bei 27,07 mol Olefin · (mmol Rh · h)⁻¹, entsprechend einer Zunahme um 2,3 Prozentpunkte, bezogen auf den Referenzwert.

### Beispiel 3

### Hydroformylierung von Cyclohexen mit Zusatz von 2-Ethylhexansäure

Entsprechend der allgemeinen Versuchsvorschrift wurde Cyclohexen in Gegenwart von 3 ppm Rhodium in Form von Rhodium-2-Ethylhexanoat zur Reaktion gebracht. In der über die externe Vorlage zugeführten Rhodiumlösung mit 2-Ethylhexanol als Lösungsmittel wurde vor dem Einleiten in den Autoklaven durch Zugabe von 2-Ethylhexansäure ein molares Verhältnis von Rhodium zu 2-Ethylhexansäure von 1:2877 eingestellt. Nach 60 Minuten lag der Cyclohexenumsatz bei 78,9%, gaschromatographisch ermittelt. Die Selektivität zu Formylcyclohexan lag bei 95,9%, gaschromatographisch ermittelt und entspricht einer Ausbeute von 75,7%, gaschromatographisch ermittelt.
Die spezifische Katalysatoraktivität lag bei 29,67 mol Olefin · (mmol Rh · h)⁻¹, entsprechend einer Zunahme um 12,1 Prozentpunkte, bezogen auf den Referenzwert.

### Beispiel 4

### Hydroformylierung von 1-Hexen mit Zusatz von 2-Ethylhexansäure

Entsprechend der allgemeinen Versuchsvorschrift wurde 1-Hexen in Gegenwart von 3 ppm Rhodium in Form von Rhodium-2-Ethylhexanoat zur Reaktion gebracht. In der über die externe Vorlage zugeführten Rhodiumlösung mit 2-Ethylhexanol als Lösungsmittel wurde vor dem Einleiten in den Autoklaven durch Zugabe von 2-Ethylhexansäure ein molares Verhältnis von Rhodium zu 2-Ethylhexansäure von 1:125 eingestellt. Nach 30 Minuten lag der 1-Hexenumsatz bei 57,1%, gaschromatographisch ermittelt. Die Aldehydselektivität zu n/i-Heptanal lag bei 62,3%, gaschromatographisch ermittelt und entspricht einer Ausbeute an n/i-Heptanalen von 35,6%, gaschromatographisch ermittelt.
Die spezifische Katalysatoraktivität lag bei 20,94 mol Olefin · (mmol Rh · h)⁻¹. Dieser Wert kann als Referenzwert mit "100" angesetzt werden.

### Beispiel 5

### Hydroformylierung von 1-Hexen mit Zusatz von 2-Ethylhexansäure

Entsprechend der allgemeinen Versuchsvorschrift wurde 1-Hexen in Gegenwart von 3 ppm Rhodium in Form von Rhodium-2-Ethylhexanoat zur Reaktion gebracht. In der über die externe Vorlage zugeführten Rhodiumlösung mit 2-Ethylhexanol als Lösungsmittel wurde vor dem Einleiten in den Autoklaven durch Zugabe von 2-Ethylhexansäure ein molares Verhältnis von Rhodium zu 2-Ethylhexansäure von 1:2877 eingestellt. Nach 30 Minuten lag der 1-Hexenumsatz bei 60,7%, gaschromatographisch ermittelt. Die Aldehydselektivität zu n/i-Heptanal lag bei 67,3%, gaschromatographisch ermittelt und entspricht einer Ausbeute an n/i-Heptanalen von 39,3%, gaschromatographisch ermittelt.
Die spezifische Katalysatoraktivität lag bei 22,26 mol Olefin · (mmol Rh · h)⁻¹, entsprechend einer Zunahme um 6,3 Prozentpunkte, bezogen auf den Referenzwert.

Wie die erfindungsgemäßen Beispiele belegen, kann überraschenderweise durch einen gezielten Zusatz an 2-Ethylhexansäure zu der der Reaktionszone zugeführten Rhodiumlösung sowohl der Umsatz an olefinisch ungesättigter Verbindung als auch die Selektivität zu den gewünschten Aldehyden in der Hydroformylierungsreaktion erhöht werden, so dass sich insgesamt die Aldehydausbeute verbessert. Soll hingegen die absolute Aldehydausbringung gleichbleiben, kann nach dem erfindungsgemäßen Verfahren der spezifische Rhodiumeinsatz verringert werden.
Trotz des Zusatzes vergleichsweiser hoher Säuremengen wird keine auffallend hohe Hochsiederbildung während der Hydroformylierungsreaktion beobachtet.

## Patentansprüche

1. Verfahren zur Herstellung von Aldehyden durch Zuführen einer organischen Lösung, die mindestens eine Rhodiumverbindung gelöst enthält, zu einer Reaktionszone und Umsetzung von olefinisch ungesättigten Verbindungen mit Kohlenmonoxid und Wasserstoff in dieser Reaktionszone in homogener organischer Phase in Gegenwart mindestens einer Rhodiumverbindung und in Abwesenheit von komplexbildenden Organophosphor-Verbindungen, **dadurch gekennzeichnet, dass** man der Rhodium haltigen organischen Lösung je Mol Rhodium mindestens eine organische Säure ausgewählt von gesättigten aliphatischen Monocarbonsäuren mit 5 bis 13 Kohlenstoffatomen im Molekül, gesättigten aliphatischen Dicarbonsäuren mit 5 bis 13 Kohlenstoffatomen im Molekül, und Sulfonsäuren mit 1 bis 12 Kohlenstoffatomen im Molekül oder Mischungen davon auf einen Gesamtgehalt, berechnet als Säureäquivalent, von mehr als 3 Mol bis 3000 Mol zusetzt und anschließend diese Lösung der Reaktionszone zuführt.

2. Verfahren gemäß Anspruch 1, **dadurch gekennzeichnet, dass** man der Rhodium haltigen organischen Lösung je Mol Rhodium die organische Säure oder Mischungen davon auf einen Gesamtgehalt, berechnet als Säureäquivalent, von 50 bis 2000 Mol und vorzugsweise von 100 bis 1000 Mol zusetzt.

3. Verfahren nach Anspruch 1 oder 2, **dadurch gekennzeichnet, dass** man als gesättigte aliphatische Monocarbonsäuren, n-Valeriansäure, 2-Methylbuttersäure, 2-Ethylhexansäure, Isononansäure, hergestellt durch Hydroformylierung von Diisobutylen und nachfolgende Oxidation des Hydroformylierungsproduktes, oder Isotridecansäure, hergestellt durch Hydroformylierung von Tetrapropylen und nachfolgende Oxidation des Hydroformylierungsproduktes, verwendet.

4. Verfahren nach Anspruch 1 oder 2, **dadurch gekennzeichnet, dass** man als gesättigte aliphatische Dicarbonsäuren Adipinsäure verwendet.

5. Verfahren nach Anspruch 1 oder 2, **dadurch gekennzeichnet, dass** man als Sulfonsäuren aliphatische, cycloaliphatische, aromatische und/oder araliphatische Sulfonsäuren verwendet.

6. Verfahren nach Anspruch 5, **dadurch gekennzeichnet, dass** man als Sulfonsäuren Methansulfonsäure, para-Toluolsulfonsäure, Benzolsulfonsäure oder Benzoldisulfonsäure verwendet.

7. Verfahren nach einem oder mehreren der Ansprüche 1 bis 6, **dadurch gekennzeichnet, dass** der Rhodiumgehalt in der der Reaktionszone zugeführten Rhodium haltigen organischen Lösung 100 bis 10000 ppm, vorzugsweise 1000 bis 10000 ppm, beträgt.

8. Verfahren nach einem oder mehreren der Ansprüche 1 bis 7, **dadurch gekennzeichnet, dass** die der Reaktionszone zugeführte Rhodium haltige organische Lösung ein polares Lösungsmittel enthält.

9. Verfahren nach Anspruch 8, **dadurch gekennzeichnet, dass** man als polares Lösungsmittel 2-Ethylhexanol oder Isononanol auf Basis von 3,5,5-Trimethylhexanol verwendet.

10. Verfahren nach einem oder mehreren der Ansprüche 1 bis 9, **dadurch gekennzeichnet, dass** in der Reaktionszone die Umsetzung der olefinisch ungesättigten Verbindungen mit Kohlenmonoxid und Wasserstoff bei Temperaturen von 20 bis 180°C, bevorzugt von 50 bis 150°C und insbesondere von 100 bis 150°C und bei Drücken von 0,1 bis 70 MPa, bevorzugt von 0,1 bis 60 MPa und insbesondere von 0,1 bis 30 MPa erfolgt.

11. Verfahren nach einem oder mehreren der Ansprüche 1 bis 10, **dadurch gekennzeichnet, dass** in der Reaktionszone die Umsetzung der olefinisch ungesättigten Verbindungen mit Kohlenmonoxid und Wasserstoff bei einem Rhodiumgehalt von 1 bis 100 ppm, vorzugsweise von 2 bis 30 ppm, jeweils bezogen auf das homogene Reaktionsgemisch, erfolgt.

12. Verfahren nach einem oder mehreren der Ansprüche 1 bis 11, **dadurch gekennzeichnet, dass** die olefinisch ungesättigten Verbindungen 2 bis 12 Kohlenstoffatome im Molekül enthalten.

## Claims

1. Process for preparing aldehydes by introduction of an organic solution containing at least one rhodium compound in dissolved form into a reaction zone and reaction of olefinically unsaturated compounds with carbon monoxide and hydrogen in a homogeneous organic phase in the presence of at least one rhodium compound and in the absence of complex-forming organophosphorus compounds in this reaction zone, **characterized in that** at least one organic acid selected from among saturated aliphatic monocarboxylic acids having from 5 to 13 carbon atoms in the molecule, saturated aliphatic dicarboxylic acids having from 5 to 13 carbon atoms in the molecule and sulphonic acids having from 1 to 12 carbon atoms in the molecule or mixture thereof in a total amount, calculated as acid equivalent, of from > 3 mol to 3000 mol per mole of rhodium is added to the rhodium-containing organic solution and this solution is subsequently fed to the reaction zone.

2. Process according to Claim 1, **characterized in that** the organic acid or mixture thereof in a total amount, calculated as acid equivalent, of from 50 mol to 2000 mol and preferably from 100 to 1000 mol per mole of rhodium is added to the rhodium-containing organic solution.

3. Process according to Claim 1 or 2, **characterized in that** n-valeric acid, 2-methylbutyric acid, 2-ethylhexanoic acid, isononanoic acid prepared by hydroformylation of diisobutylene and subsequent oxidation of the hydroformylation product or isotridecanoic acid prepared by hydroformylation of tetrapropylene and subsequent oxidation of the hydroformylation product is used as saturated aliphatic monocarboxylic acid.

4. Process according to Claim 1 or 2, **characterized in that** adipic acid is used as saturated aliphatic dicarboxylic acid.

5. Process according to Claim 1 or 2, **characterized in that** aliphatic, cycloaliphatic, aromatic and/or araliphatic sulphonic acids are used as sulphonic acids.

6. Process according to Claim 5, **characterized in that** methanesulphonic acid, para-toluenesulphonic acid, benzenesulphonic acid or benzenedisulphonic acid is used as sulphonic acid.

7. Process according to one of more of Claims 1 to 6, **characterized in that** the rhodium content of the rhodium-containing organic solution fed to the reaction zone is from 100 to 10 000 ppm, preferably from 1000 to 10 000 ppm.

8. Process according to one or more of Claims 1 to 7, **characterized in that** the rhodium-containing organic solution fed to the reaction zone contains a polar solvent.

9. Process according to Claim 8, **characterized in that** 2-ethylhexanol or isononanol based on 3,5,5-trimethylhexanol is used as polar solvent.

10. Process according to one or more of Claims 1 to 9, **characterized in that** the reaction of the olefinically unsaturated compounds with carbon monoxide and hydrogen in the reaction zone is carried out at temperatures of from 20 to 180°C, preferably from 50 to 150°C and in particular from 100 to 150°C, and pressures of from 0.1 to 70 MPa, preferably from 0.1 to 60 MPa and in particular from 0.1 to 30 MPa.

11. Process according to one or more of Claims 1 to 10, **characterized in that** the reaction of the olefinically unsaturated compounds with carbon monoxide and hydrogen in the reaction zone is carried out at a rhodium content of from 1 to 100 ppm, preferably from 2 to 30 ppm, in each case based on the homogeneous reaction mixture.

12. Process according to one or more of Claims 1 to 11, **characterized in that** the olefinically unsaturated compounds contain from 2 to 12 carbon atoms in the molecule.

## Revendications

1. Procédé de fabrication d'aldéhydes par introduction d'une solution organique, qui contient au moins un composé de rhodium dissous, dans une zone de réaction et mise en réaction de composés oléfiniquement insaturés avec du monoxyde de carbone et de l'hydrogène dans cette zone de réaction en phase organique homogène en présence d'au moins un composé de rhodium et en l'absence de composés d'organophosphore formant des complexes, **caractérisé en ce qu'**au moins un acide organique choisi parmi les acides monocarboxyliques aliphatiques saturés de 5 à 13 atomes de carbone par molécule, les acides dicarboxyliques aliphatiques saturés de 5 à 13 atomes de carbone par molécule et les acides sulfoniques de 1 à 12 atomes de carbone par molécule ou leurs mélanges est ajouté à la solution organique contenant du rhodium par mole de rhodium en une teneur totale, calculée sous forme d'équivalent d'acide, de plus de 3 moles à 3 000 moles, puis cette solution est introduite dans la zone de réaction.

2. Procédé selon la revendication 1, **caractérisé en ce que** l'acide organique ou des mélanges de ceux-ci sont ajoutés à la solution organique contenant du rhodium par mole de rhodium en une teneur totale, calculée sous forme d'équivalent d'acide, de 50 à 2 000 moles, et de préférence de 100 à 1 000 moles.

3. Procédé selon la revendication 1 ou 2, **caractérisé en ce que** de l'acide n-valérique, de l'acide 2-méthylbutyrique, de l'acide 2-éthylhexanoïque, de l'acide isononanoïque, fabriqué par hydroformylation de diisobutylène et oxydation ultérieure du produit d'hydroformylation, ou de l'acide isotridécanoïque, fabriqué par hydroformylation de tétrapropylène et oxydation ultérieure du produit d'hydroformylation, est utilisé en tant qu'acides monocarboxyliques aliphatiques saturés.

4. Procédé selon la revendication 1 ou 2, **caractérisé en ce que** de l'acide adipique est utilisé en tant qu'acides dicarboxyliques aliphatiques saturés.

5. Procédé selon la revendication 1 ou 2, **caractérisé en ce que** des acides sulfoniques aliphatiques, cycloaliphatiques, aromatiques et/ou araliphatiques sont utilisés en tant qu'acides sulfoniques.

6. Procédé selon la revendication 5, **caractérisé en ce que** de l'acide méthanesulfonique, de l'acide paratoluènesulfonique, de l'acide benzènesulfonique ou de l'acide benzènedisulfonique est utilisé en tant qu'acides sulfoniques.

7. Procédé selon une ou plusieurs des revendications 1 à 6, **caractérisé en ce que** la teneur en rhodium dans la solution organique contenant du rhodium introduite dans la zone de réaction est de 100 à 10 000 ppm, de préférence de 1 000 à 10 000 ppm.

8. Procédé selon une ou plusieurs des revendications 1 à 7, **caractérisé en ce que** la solution organique contenant du rhodium introduite dans la zone de réaction contient un solvant polaire.

9. Procédé selon la revendication 8, **caractérisé en ce que** du 2-éthylhexanol ou de l'isononanol à base de 3,5,5-triméthylhexanol est utilisé en tant que solvant polaire.

10. Procédé selon une ou plusieurs des revendications 1 à 9, **caractérisé en ce que** la mise en réaction des composés oléfiniquement insaturés avec du monoxyde de carbone et de l'hydrogène dans la zone de réaction a lieu à des températures de 20 à 180 °C, de préférence de 50 à 150 °C, et notamment de 100 à 150 °C, et à des pressions de 0,1 à 70 MPa, de préférence de 0,1 à 60 MPa, et notamment de 0,1 à 30 MPa.

11. Procédé selon une ou plusieurs des revendications 1 à 10, **caractérisé en ce que** la mise en réaction des composés oléfiniquement insaturés avec du monoxyde de carbone et de l'hydrogène dans la zone de réaction a lieu à une teneur en rhodium de 1 à 100 ppm, de préférence de 2 à 30 ppm, à chaque fois par rapport au mélange réactionnel homogène.

12. Procédé selon une ou plusieurs des revendications 1 à 11, **caractérisé en ce que** les composés oléfiniquement insaturés contiennent 2 à 12 atomes de carbone par molécule.
